# EUROPEAN PATENT SPECIFICATION

(11) **EP 1 635 830 B1**
(45) Date of publication and mention of the grant of the patent: **05.11.2008**
(21) Application number: 03754672.8
(22) Date of filing: 15.09.2003
(51) Int. Cl.: A61K 9/20

(54) **GRANULATE FORMULATION OF THE RAPAMYCIN ESTER CCI-779**
GRANULIERTE FORMULIERUNG DES RAPAMYCINESTERS CCl-779
COMPOSITION GRANULEE D'UN ESTER DE RAPAMYCINE CCl-779

(30) Priority: 17.09.2002 US 411264 P
(43) Date of publication of application: 22.03.2006
(73) Proprietor: Wyeth, Madison, New Jersey 07940 (US)
(72) Inventor: ASHRAF, Muhammad, Elmwood Park, NJ 07407 (US); BENJAMIN, Eric, J., Jamestown, NC 27282 (US)
(74) Representative: Wileman, David Francis
(86) International application number: PCT/US2003/029228
(87) International publication number: WO 2004/026280

(56) References cited:
- US-A- 5 362 718
- US-A1- 2002 091 137

## Description

### BACKGROUND OF THE INVENTION

This invention relates to oral solid formulations of rapamycin 42-ester with 3-hydroxy-2-(hydroxymethyl)-2-methylpropionic acid (CCI-779).

Rapamycin is a macrocyclic triene antibiotic produced by *Streptomyces hygroscopicus,* which was found to have antifungal activity, particularly against Candida *albicans,* both *in vitro* and *in vivo* [C. Vezina et al., J. Antibiot. 28, 721 (1975); S.N. Sehgal et al., J. Antibiot. 28, 727 (1975); H. A. Baker et al., J. Antibiot. 31, 539 (1978); U.S. Patent 3,929,992; and U.S. Patent 3,993,749]. Additionally, rapamycin alone (U.S. Patent 4,885,171) or in combination with picibanil (U.S. Patent 4,401,653) has been shown to have antitumor activity.

The immunosuppressive effects of rapamycin have been disclosed in FASEB 3, 3411 (1989). Cyclosporin A and FK-506, other macrocyclic molecules, also have been shown to be effective as immunosuppressive agents, therefore useful in preventing transplant rejection [U.S. Patent 5,100,899]. R. Martel et a/. [Can. J. Physiol. Pharmacol. 55, 48 (1977)] disclosed that rapamycin is effective in the experimental allergic encephalomyelitis model, a model for multiple sclerosis; in the adjuvant arthritis model, a model for rheumatoid arthritis; and effectively inhibited the formation of IgE-like antibodies.

Rapamycin is also useful in preventing or treating systemic lupus erythematosus [U.S. Patent 5,078,999], pulmonary inflammation [U.S. Patent 5,080,899], insulin dependent diabetes mellitus [U.S. Patent 5,321,009], skin disorders, such as psoriasis [U.S. Patent 5,286,730], bowel disorders [U.S. Patent 5,286,731], smooth muscle cell proliferation and intimal thickening following vascular injury [U.S. Patents 5,288,711 and 5,516,781], adult T-cell leukemia/lymphoma [European Patent Application 525,960 A1], ocular inflammation [U.S. Patent 5,387,589], malignant carcinomas [U.S. Patent 5,206,018], cardiac inflammatory disease [U.S. Patent 5,496,832], and anemia [U.S. Patent 5,561,138].

Rapamycin 42-ester with 3-hydroxy-2-(hydroxymethyl)-2-methylpropionic acid (CCI-779) is ester of rapamycin which has demonstrated significant inhibitory effects on tumor growth in both in vitro and in vivo models. The preparation and use of hydroxyesters of rapamycin, including CCI-779, are disclosed in U.S. Patent 5,362,718.

CCI-779 exhibits cytostatic, as opposed to cytotoxic properties, and may delay the time to progression of tumors or time to tumor recurrence. CCI-779 is considered to have a mechanism of action that is similar to that of sirolimus. CCI-779 binds to and forms a complex with the cytoplasmic protein FKBP, which inhibits an enzyme, mTOR (mammalian target of rapamycin, also known as FKBP12-rapamycin associated protein [FRAP]). Inhibition of mTOR's kinase activity inhibits a variety of signal transduction pathways, including cytokine-stimulated cell proliferation, translation of mRNAs for several key proteins that regulate the G1 phase of the cell cycle, and IL-2-induced transcription, leading to inhibition of progression of the cell cycle from G1 to S. The mechanism of action of CCI-779 that results in the G1 to S phase block is novel for an anticancer drug.

*In vitro,* CCI-779 has been shown to inhibit the growth of a number of histologically diverse tumor cells. Central nervous system (CNS) cancer, leukemia (T-cell), breast cancer, prostate cancer, and melanoma lines were among the most sensitive to CCI-779. The compound arrested cells in the G1 phase of the cell cycle.

*In vivo* studies in nude mice have demonstrated that CCI-779 has activity against human tumor xenografts of diverse histological types. Gliomas were particularly sensitive to CCI-779 and the compound was active in an orthotopic glioma model in nude mice. Growth factor (platelet-derived)-induced stimulation of a human glioblastoma cell line in vitro was markedly suppressed by CCI-779. The growth of several human pancreatic tumors in nude mice as well as one of two breast cancer lines studied in vivo also was inhibited by CCI-779. US 2002/091137 discloses its use as antineoplastic agent.

One obstacle towards the formulation of CCI-779 is its poor aqueous solubility (less than 1 µg/ml), which makes its bioavailability low. In additional, CCI-779 exhibits aqueous instability via cleavage of a lactone bond, resulting in the formation of the ring opened seco-CCI-779. CCI-779 tablets prepared by direct compression of non-micronized CCI-779 with standard excipients and fillers, in the presence or absence of surfactants provided tablets which did not exhibit rapid and complete drug release, and thereby provided an unsuitable formulation for CCI-779.

### SUMMARY OF THE INVENTION

This invention avoids the aforementioned problems by employing a water-soluble polymer such as povidone (PVP) and employing a wet granulation process to provide a highly bioavailable non-micronized CCI-779 formulation that overcomes the dissolution and instability problem. The inhibition of degradation can also be assisted by the use of one or more antioxidants, and a pH modifying agent to maintain a pH of about 4 to about 6.

Other aspects and advantages of the invention will be apparent from the following detailed description.

### DETAILED DESCRIPION OF THE INVENTION

Accordingly, this invention provides a solid formulation comprising a granulation prepared using a wet granulation process, said granulation comprising CCI-779, a water soluble polymer, a pH modifying agent, a surfactant, and an antioxidant. In one embodiment, the formulation contains from 0.1 to 30%, from 0.5 to 25%, from 1 to 20%, from 5 to 15%, or from 7 to 12% (wt/wt) CCI-779, from 0.5 to 50%, from 1 to 40%, from 5 to 35%, from 10 to 25%, or from 15 to 20% (wt/wt) water soluble polymer, from 0.5 to 10%, 1 to 8%, or 3 to 5% (wt/wt) surfactant, and from 0.001% to 1%, 0.01% to 1%, or 0.1% to 0.5% (wt/wt) antioxidant. However, other embodiments may contain more, or less, of these components.

The formulation may also contain suitable chelating agents, fillers, binders, surfactants, and the like to facilitate the granulation and tableting process. It is preferred that the wet granulation be performed with a hydroalcoholic solvent system comprising water and an alcohol, with ethanol being the preferred alcoholic component.

Typical water soluble polymers include, but are not limited to, polyvinylpyrrolidone (PVP), hydroxypropylmethylcellulose (HPMC), polyethylene glycol (PEG), and cyclodextrin or mixtures thereof. It is preferred that the water-soluble polymer is PVP, and having a molecular weight of between 2.5 and 60 kilodaltons. Any given formulation of this invention may contain multiple ingredients of each class of component. For example, a formulation containing an antioxidant may contain one or more antioxidants as the antioxidant component.

Acceptable pH modifying agents include, but are not limited to citric acid, sodium citrate, dilute HCl, and other mild acids or bases capable of buffering a solution containing CCI-779 to a pH in the range of about 4 to about 6.

Acceptable antioxidants include, but are not limited to, citric acid, d,l-α-tocopherol, BHA, BHT, monothioglycerol, ascorbic acid, and propyl gallate. It is expected that the antioxidants of the formulations of this invention will be used in concentrations ranging from 0.001 % to 3% wt/wt.

Chelating agents, and other materials capable of binding metal ions, such as ethylene diamine tetra acetic acid (EDTA) and its salts are capable of enhancing the stability of CCI-779.

Surfactants may include polysorbate 80, sodium lauryl sulfate, sodium dodecyl sulfate, salts of bile acids (taurocholate, glycocholate, cholate, deoxycholate, etc.) that may be combined with lecithin. Alternatively, ethoxylated vegetable oils, such as Cremophor EL, vitamin E tocopherol propylene glycol succinate (Vitamin E TGPS), polyoxyethylene-polyoxypropylene block copolymers, and poloxamers.

Binders, fillers, and disintegrants such as sucrose, lactose, microcrystalline cellulose, croscarmellose sodium, magnesium stearate, gum acacia, cholesterol, tragacanth, stearic acid, gelatin, casein, lecithin (phosphatides), carboxymethylcellulose calcium, carboxymethylcellulose sodium, methylcellulose, hydroxyethylcellulose, hydroxypropylcellulose, hydroxypropylmethycellulose phthalate, noncrystalline cellulose, cetostearyl alcohol, cetyl alcohol, cetyl esters wax, dextrates, dextrin, lactose, dextrose, glyceryl monooleate, glyceryl monostearate, glyceryl palmitostearate, polyoxyethylene alkyl ethers, polyethylene glycols, polyoxyethylene castor oil derivatives, polyoxyethylene stearates, and polyvinyl alcohol, and the like may also be incorporated into the formulation.

The formulation can be prepared by preparing an alcoholic solution comprising CCI-779 and an antioxidant, and an aqueous solution comprising a water-soluble polymer, a surfactant, and a pH modifier, in sufficient quantity to adjust the pH of the aqueous solution to 4 to 6. Suitable alcohols include methanol, ethanol, isopropanol, and the like, where ethanol is the preferred alcohol. The solutions were mixed and added to a mixer containing intragranular excipients. Alternatively, the alcoholic and aqueous solutions can be added separately without mixing with each other. Such intragranular excipients comprise binders and fillers to promote dissolution enhancement. Typical intragranular excipients may include, but are not limited to, microcrystalline cellulose, lactose, and croscarmellose sodium. The solid intragranular excipients are granulated with the solutions in the mixer until a uniform granulation is achieved. The mixer can be a blender with intensifying bar, a low shear granulator or a high shear granulator. The granulation is dried in a fluid bed dryer at approximately 50°C, and milled using a suitable milling device, such as a Fitz mill. The wet granulation and drying can be done in a fluid bed granulator/dryer. The wet granulation can be dried using an tray drying oven. If desired, the dried granulation can be further blended with extragranular fillers and binders, such as microcrystalline cellulose, croscarmellose sodium, and magnesium stearate in a blender, such as a V-blender, before compression into tablets.

Alternatively, some of the water-soluble polymer can be contained in the intragranular excipients, and the aqueous and alcoholic solutions added to the mixer containing the intragranular excipients stepwise. For example, the order of addition to the mixer may be one half of the aqueous solution, followed by the entire alcoholic solution, and then the remainder of the aqueous solution. Other sequences of addition are possible and permissible under this invention.

The following provide representative examples of the formulations of this invention. The preparation of CCI-779 is described in U.S. Patent 5,362,718. A regioselective preparation of CCI-779 is described in US Patent 6,277,983. These examples are illustrative only, and do not limit the invention.

### EXAMPLES

### Procedure A

The following procedure was used to prepare a tablet containing 2 mg CCI-779 containing the following components; quantities are adjusted to account for low potency:

| Ingredient | Percent Wt/Wt |
|---|---|
| CCI-779 | 1.77 |
| Butylated Hydroxyanisole | 0.10 |
| Butylated Hydroxytoluene | 0.05 |
| PVP, 17PF | 8.85 |
| Edetic Acid | 0.01 |
| Sodium Lauryl Sulfate | 3.0 |
| Sodium Citrate (anhydrous) | 0.75 |
| Citric Acid (anhydrous) | 0.25 |
| Microcrystalline Cellulose | 50.4 |
| Croscarmellose Sodium | 4.0 |
| Anhydrous Lactose | 30.32 |
| Magnesium Stearate | 0.5 |
| Dehydrated Alcohol (ethanol)* | |
| Purified Water* | |

| | |
|---|---|
| * Used in processing, but does not appear in final product. | |

Microcrystalline cellulose, anhydrous lactose, and croscarmellose sodium were screened through a 20 mesh screen, transferred to a V-Blender with an intensifying bar and mixed. Sodium lauryl sulfate, edetic acid, sodium citrate, citric acid, and PVP were dissolved in a sufficient quantity of purified water to achieve a solution. Butylated hydroxyanisole, butylated hydroxytoluene, and CCI-779 were dissolved in a sufficient quantity of dehydrated alcohol to achieve a solution. The alcohol solution was added to the aqueous solution with stirring. The alcoholic solution container was washed with dehydrated alcohol, which was added to the hydroholic solution. The solution was stirred until a clear solution resulted. The hydroholic solution was added to the V-Blender and the ingredients granulated. The hydroholic solution container was washed with a 10% alcohol solution that was added to the granulation. The granulation was mixed until uniformity was achieved, followed by drying in a fluid bed dryer. The dried granulation was passed through a 30 mesh screen, and any oversized granulation milled through a Fitzmill. The milled granulation was transferred to a V-Blender. Additional microcrystalline cellulose, anhydrous lactose, and croscarmellose sodium were passed through a 20 mesh screen and added to the blender. The mixture was blended, magnesium stearate (screened through a 30 mesh screen) was added to the blender, and the mixture blended. The resulting mixture was compressed into tablets.

The following table shows a comparison of the dissolution in water of (a) pure CCI-779 in capsules, (b) a tablet of the dry blend of the same ingredients contained in the granulation, and (c) a tablet of the granulation prepared as described above. The results clearly demonstrate that the hydroholic granulation of this invention provided enhanced dissolution in water, and will thereby provide enhanced bioavailability.

| | Percent CCI-779 Dissolved | | |
|---|---|---|---|
| Time (min) | CCI-779 Capsules | CCI-779 Dry Blend Tablet | CCI-779 Wet Granulation |
| 10 | 4 | 31 | 96 |
| 20 | 9 | 42 | 104 |
| 30 | 14 | 50 | 104 |
| 45 | 21 | 56 | 104 |

### Procedure B

The following procedure was used to prepare a tablet containing 25 mg CCI-779 containing the following components; quantities are adjusted to account for low potency:

| Ingredient | Percent Wt/Wt |
|---|---|
| CCI-779 | 4.0 |
| Butylated Hydroxyanisole | 0.10 |
| Butylated Hydroxytoluene | 0.05 |
| PVP, 17PF | 21.0 |
| Edetic Acid | 0.01 |
| Sodium Lauryl Sulfate | 3.6 |
| Citric Acid (anhydrous) | 0.025 |
| Microcrystalline Cellulose | 44.5 |
| Croscarmellose Sodium | 4.0 |
| Anhydrous Lactose | 22.17 |
| Magnesium Stearate | 0.5 |
| Dehydrated Alcohol (ethanol)* | |
| Purified Water* | |

| | |
|---|---|
| * Used in processing, but does not appear in final product. | |

Microcrystalline cellulose, anhydrous lactose, approximately one half of the PVP, and croscarmellose sodium were screened through a 20 mesh screen, transferred to a V-Blender with an intensifying bar and mixed. Sodium lauryl sulfate, edetic acid, citric acid, and the remaining PVP were dissolved in a sufficient quantity of purified water to achieve a solution. The pH of the solution was measured, and if higher than 4.5, it was lowered with 0.1 N HCl until a pH of 4.5 was achieved. Butylated hydroxyanisole, butylated hydroxytoluene, and CCI-779 were dissolved in a sufficient quantity of dehydrated alcohol to achieve a solution. About one half of the aqueous solution was added to the blender, and the granulation mixed for about 4 minutes. The alcoholic solution was added to the blender and the granulation mixed for about 4 minutes. The remaining aqueous solution was added to the blender and the granulation mixed for about 4 minutes. Additional water was added, if needed to make a uniform granulation. The granulation was dried in a fluid bed dryer at a temperature of about 50°C. The dried granulation was passed through a 30 mesh screen, and any oversized granulation milled through a Fitzmill. The milled granulation was transferred to a V-Blender. Additional microcrystalline cellulose, anhydrous lactose, and croscarmellose sodium were passed through a 20 mesh screen and added to the blender. The mixture blended and magnesium stearate (screened through a 30 mesh screen) was added to the blender, and the mixture blended. The resulting mixture was compressed into tablets.

## Claims

1. A pharmaceutical composition for oral administration comprising a granulation, said granulation comprising CCI-779, a water soluble polymer, a surfactant, an antioxidant from 0.001% to 3% (wt/wt), and a pH modifying agent.

2. The composition of claim 1, wherein the water soluble polymer is PVP, hydroxypropylmethylcellulose, polyethylene glycol, or cyclodextrin or mixtures thereof.

3. The composition of claim 2, wherein the water soluble polymer is PVP.

4. The composition of any of claims 1 to 3, wherein the surfactant is polysorbate 80, sodium lauryl sulfate, sodium dodecyl sulfate, a salt of a bile acid, an ethoxylated vegetable oil, a polyoxyethylene-polyoxypropylene block copolymer, or a poloxamer.

5. The composition of claim 4, wherein the surfactant is sodium lauryl sulfate or sodium dodecyl sulfate.

6. The pharmaceutical composition of any of claims 1 to 5, wherein the pH modifying agent is selected from the group consisting of sodium citrate, citric acid, dilute hydrochloric acid, and mixtures thereof.

7. The composition of any of claims 1 to 6, wherein the antioxidant is butylated hydroxyanisole, or butylated hydroxytoluene.

8. A process for preparing a CCI-779 oral composition, which comprises
(a) dissolving CCI-779 and from 0.001% to 3%(wt/wt) of an antioxidant in an alcohol;
(b) dissolving PVP, a pH modifying agent, and a surfactant in water;
(c) combining the aqueous and alcoholic solutions to provide a hydroholic solution;
(d) adding the hydroholic solution to a mixer containing one or more intragranular excipients;
(e) granulating the mixture; and
(f) drying the resulting granulation.

9. The process of claim 8, wherein the pH modifying agent is selected from the group consisting of citric acid, sodium citrate, hydrochloric acid and mixtures thereof.

10. The process of claim 9, wherein the alcohol is ethanol.

11. The process of any of claims 8 to 10, wherein the antioxidant is butylated hydroxyanisole or butylated hydroxytoluene.

12. The process of any of claims 8 to 11, wherein the surfactant is sodium lauryl sulfate.

13. A process for preparing a CCI-779 oral formulation comprising
(a) dissolving CCI-779 and from 0.001% to 3% (wt/wt) of an antioxidant in an alcohol;
(b) dissolving PVP, a pH modifying agent, and a surfactant in water;
(c) adding the aqueous and alcoholic solutions stepwise, and in one or more portions each, to a mixer containing one or more intragranular excipients;
(e) granulating the mixture; and
(f) drying the resulting granulation.

14. The process of claim 13, wherein the pH modifying agent is selected from the group consisting of citric acid, sodium citrate, hydrochloric acid and mixtures thereof.

15. The process of claim 14, wherein the alcohol is ethanol.

16. The process of any of claims 13 to 15, wherein the antioxidant is butylated hydroxyanisole and butylated hydroxytoluene.

17. The process of claim 16, wherein the surfactant is sodium lauryl sulfate.

## Patentansprüche

1. Pharmazeutische Zusammensetzung zur oralen Verabreichung, die eine Granulierung umfasst, wobei die Granulierung CCI-779, ein wasserlösliches Polymer, einen oberflächenaktiven Stoff, ein Antioxidationsmittel von 0,001 Gew.-% bis 3 Gew.-% und ein den pH-Wert modifizierendes Mittel umfasst.

2. Zusammensetzung nach Anspruch 1, wobei es sich bei dem wasserlöslichen Polymer um PVP, Hydroxypropylmethylcellulose, Polyethylenglykol oder Cyclodextrin oder Gemische davon handelt.

3. Zusammensetzung nach Anspruch 2, wobei es sich bei dem wasserlöslichen Polymer um PVP handelt.

4. Zusammensetzung nach einem der Ansprüche 1 bis 3, wobei es sich bei dem oberflächenaktiven Stoff um Polysorbat 80, Natriumlaurylsulfat, Natriumdodecylsulfat, ein Salz einer Gallensäure, ein ethoxyliertes pflanzliches Öl, ein Polyoxyethylen/Polyoxypropylen-Blockcopolymer oder ein Poloxamer handelt.

5. Zusammensetzung nach Anspruch 4, wobei es sich bei dem oberflächenaktiven Stoff um Natriumlaurylsulfat oder Natriumdodecylsulfat handelt.

6. Pharmazeutische Zusammensetzung nach einem der Ansprüche 1 bis 5, wobei das den pH-Wert modifizierende Mittel aus der Gruppe bestehend aus Natriumcitrat, Citronensäure, verdünnter Salzsäure und Gemischen davon ausgewählt ist.

7. Zusammensetzung nach einem der Ansprüche 1 bis 6, wobei es sich bei dem Antioxidationsmittel um Butylhydroxyanisol oder Butylhydroxytoluol handelt.

8. Verfahren zum Herstellen einer oralen CCI-779-Zusammensetzung, das Folgendes umfasst:
(a) Lösen von CCI-779 und von 0,001 Gew.-% bis 3 Gew.-% eines Antioxidationsmittels in einem Alkohol;
(b) Lösen von PVP, einem den pH-Wert modifizierenden Mittel und einem oberflächenaktiven Stoff in Wasser;
(c) Vereinen der wässrigen und der alkoholischen Lösung, um eine wässrigalkoholische Lösung bereitzustellen;
(d) Geben der wässrig-alkoholischen Lösung in ein Mischgerät, das ein oder mehrere intragranuläre Vehikel enthält;
(e) Granulieren des Gemischs und
(f) Trocknen der resultierenden Granulierung.

9. Verfahren nach Anspruch 8, wobei das den pH-Wert modifizierende Mittel aus der Gruppe bestehend aus Citronensäure, Natriumcitrat, Salzsäure und Gemischen davon ausgewählt ist.

10. Verfahren nach Anspruch 9, wobei es sich bei dem Alkohol um Ethanol handelt.

11. Verfahren nach einem der Ansprüche 8 bis 10, wobei es sich bei dem Antioxidationsmittel um Butylhydroxyanisol oder Butylhydroxytoluol handelt.

12. Verfahren nach einem der Ansprüche 8 bis 11, wobei es sich bei dem oberflächenaktiven Stoff um Natriumlaurylsulfat handelt.

13. Verfahren zum Herstellen einer oralen CCI-779-Zusammensetzung, das Folgendes umfasst:
(a) Lösen von CCI-779 und von 0,001 Gew.-% bis 3 Gew.-% eines Antioxidationsmittels in einem Alkohol;
(b) Lösen von PVP, einem den pH-Wert modifizierenden Mittel und einem oberflächenaktiven Stoff in Wasser;
(c) Geben der wässrigen und der alkoholischen Lösung, schrittweise und in jeweils einer oder mehreren Portionen, in ein Mischgerät, das ein oder mehrere intragranuläre Vehikel enthält;
(e) Granulieren des Gemischs und
(f) Trocknen der resultierenden Granulierung.

14. Verfahren nach Anspruch 13, wobei das den pH-Wert modifizierende Mittel aus der Gruppe bestehend aus Citronensäure, Natriumcitrat, Salzsäure und Gemischen davon ausgewählt ist.

15. Verfahren nach Anspruch 14, wobei es sich bei dem Alkohol um Ethanol handelt.

16. Verfahren nach einem der Ansprüche 13 bis 15, wobei es sich bei dem Antioxidationsmittel um Butylhydroxyanisol oder Butylhydroxytoluol handelt.

17. Verfahren nach Anspruch 16, wobei es sich bei dem oberflächenaktiven Stoff um Natriumlaurylsulfat handelt.

## Revendications

1. Composition pharmaceutique pour une administration par voie orale, comprenant un produit de granulation, ledit produit de granulation comprenant CCI-779, un polymère soluble dans l'eau, un agent tensioactif, un antioxydant à concurrence de 0,001 % en poids à 3 % en poids et un agent modificateur du pH.

2. Composition selon la revendication 1, dans laquelle le polymère soluble dans l'eau est de la PVP, de l'hydroxypropylméthylcellulose, du polyéthylène glycol ou de la cyclodextrine, ou bien leurs mélanges.

3. Composition selon la revendication 2, dans laquelle le polymère soluble dans l'eau est de la PVP.

4. Composition selon l'une quelconque des revendications 1 à 3, dans laquelle l'agent tensioactif est du Polysorbate 80, du laurylsulfate de sodium, du dodécylsulfate de sodium, un sel d'un acide biliaire, une huile végétale éthoxylée, un copolymère séquencé de polyoxyéthylène-polyoxypropylène, ou un poloxamère.

5. Composition selon la revendication 4, dans laquelle l'agent tensioactif est du laurylsulfate de sodium ou du dodécylsulfate de sodium.

6. Composition pharmaceutique selon l'une quelconque des revendications 1 à 5, dans laquelle l'agent modificateur du pH est choisi parmi le groupe constitué par le citrate de sodium, l'acide citrique, de l'acide chlorhydrique dilué, et leurs mélanges.

7. Composition selon l'une quelconque des revendications 1 à 6, dans laquelle l'antioxydant est de l'hydroxyanisole butylé ou de l'hydroxytoluène butylé.

8. Procédé pour préparer une composition orale de CCI-779, qui comprend
(a) la dissolution dans un alcool de CCI-779 et d'un antioxydant, à concurrence de 0,001 % en poids à 3 % en poids ;
(b) la dissolution de PVP, d'un agent modificateur du pH et d'un agent tensioactif dans de l'eau ;
(c) la combinaison des solutions aqueuse et alcoolique pour obtenir une solution hydroalcoolique ;
(d) addition de la solution hydroalcoolique à un mélangeur contenant un ou plusieurs excipients intragranulaires ;
(e) la granulation du mélange ; et
(f) le séchage du produit de granulation résultant.

9. Procédé selon la revendication 8, dans lequel l'agent modificateur du pH est choisi parmi le groupe constitué par l'acide citrique, le citrate de sodium, l'acide chlorhydrique et leurs mélanges.

10. Procédé selon la revendication 9, dans lequel l'alcool est de l'éthanol.

11. Procédé selon l'une quelconque des revendications 8 à 10, dans lequel l'antioxydant est de hydroxyanisole butylé ou de l'hydroxytoluène butylé.

12. Procédé selon l'une quelconque des revendications 8 à 11, dans lequel l'agent tensioactif est du laurylsulfate de sodium.

13. Procédé pour préparer une formulation orale de CCI-779, comprenant :
(a) la dissolution dans un alcool de CCI-779 et d'un antioxydant, à concurrence de 0,001 % en poids à 3 % en poids;
(b) la dissolution de PVP, d'un agent modificateur du pH et d'un agent tensioactif dans de l'eau ;
(c) l'addition progressive des solutions aqueuse et alcoolique, et en une ou plusieurs portions respectivement, à un mélangeur contenant un ou plusieurs excipients intragranulaires ;
(e) la granulation du mélange ; et
(f) le séchage du produit de granulation résultant.

14. Procédé selon la revendication 13, dans lequel l'agent modificateur du pH est choisi parmi le groupe constitué par l'acide citrique, le citrate de sodium, l'acide chlorhydrique et leurs mélanges.

15. Procédé selon la revendication 14, dans lequel l'alcool est de l'éthanol.

16. Procédé selon l'une quelconque des revendications 13 à 15, dans lequel l'antioxydant est de hydroxyanisole butylé ou de l'hydroxytoluène butylé.

17. Procédé selon la revendication 16, dans lequel l'agent tensioactif est le laurylsulfate de sodium.
